# EUROPÄISCHE PATENTSCHRIFT

(11) **EP 1 912 688 B1**
(45) Veröffentlichungstag und Bekanntmachung des Hinweises auf die Patenterteilung: **18.04.2012**
(21) Anmeldenummer: 06775759.1
(22) Anmeldetag: 26.07.2006
(51) Int. Cl.: A61M 1/34, A61L 2/07, A61M 1/02

(54) **DAMPFSTERILISIERBARE BLUTTRENNVORRICHTUNG**
VAPOUR-STERISABLE BLOOD SEPARATING DEVICE
DISPOSITIF DE SEPARATION DU SANG STERILISABLE A LA VAPEUR

(30) Priorität: 26.07.2005 DE 102005035528
(43) Veröffentlichungstag der Anmeldung: 23.04.2008
(73) Patentinhaber: Hemacon GmbH, 40221 Düsseldorf (DE)
(72) Erfinder: HEIM, Gerd, H., 45964 Gladbeck (DE)
(74) Vertreter: Kreuzkamp, Markus
(86) Internationale Anmeldenummer: PCT/DE2006/001308
(87) Internationale Veröffentlichungsnummer: WO 2007/012321

(56) Entgegenhaltungen:
- EP-A- 1 671 664
- DE-U1- 20 014 311
- US-A1- 2005 051 486

## Beschreibung

Die Erfindung richtet sich auf eine Vorrichtung zur Auftrennung von Blut in einzelne und/oder Gruppen seiner Bestandteile gemäß dem Oberbegriff des Anspruches 1. Die Vorrichtung in Form eines Systems umfasst als zusammengebaute Gesamtvorrichtung eine Filteranordnung, die mittels darin angeordneter Filterelemente eine Einlasskammer 4 und eine Auslasskammer 5 ausbildet, einen Aufnahmebehälter 6 für zelluläre Blutbestandteile und einen Aufnahmebehälter 7 für Blutplasma, zu oder von der Filteranordnung 2 führende, schlauchförmige Leitungen aus Kunststoffschlauch, wobei die Einlasskammer 4 der Filteranordnung eingangsseitig mit einer Blutquelle leitungsmäßig verbindbar ist, die Einlasskammer 4 ausgangsseitig mit dem Aufnahmebehälter 6 für zelluläre Bestandteile des Blutes leitungsmäßig verbunden ist und die Auslasskammer 5 ausgangsseitig mit dem Aufnahmebehälter 7 für Blutplasma leitungsmäßig verbunden ist.

Weiterhin richtet sich die Erfindung auf ein Verfahren zur Dampfsterilisation einer Vorrichtung, insbesondere der vorbenannten, gattungsgemäßen Art, zur Auftrennung von Blut in einzelne und/oder Gruppen seiner Bestandteile in Form eines sterilisierbaren Systems, welches als zusammenzubauende Gesamtvorrichtung eine Filteranordnung, die mittels darin angeordneter Filterelemente eine Einlasskammer 4 und eine Auslaßkammer 5 ausbildet, einen Aufnahmebehälter 6 für zelluläre Blutbestandteile und einen Aufnahmebehälter 7 für Blutplasma und Leitungen aus Kunststoffschlauch umfasst, wobei die Vorrichtung als System zusammengebaut und zusammengestellt wird, wobei wiederum die Einlasskammer 4 der Filteranordnung ausgangsseitig mit einem Aufnahmebehälter 6 für zelluläre Bestandteile des Blutes leitungsmäßig verbunden wird und wobei die Auslasskammer 5 ausgangsseitig mit einem Aufnahmebehälter 7 für Blutplasma leitungsmäßig verbunden wird.

### Technischer Hintergrund

Aus der US2005-051486 A1 ist eine einfache Blutreinigungsvorrichtung bekannt, welche in einer linearen Anordnung ein Blutvolumen schwerkraftunterstützt über einen Filter reinigt und ohne wesentlichen Volumenverlust in ein Auffangbehältnis überführt. Kaskaden mehrerer Filter sind hierbei parallel oder in Reihe möglich. In jedem Fall werden die zu entfernenden Stoffe über einen Filter abgetrennt, während die Flüssigkeit durchgeleitet wird. Um eine vollständige Entleerung des QuellBehälters sicherzustellen, sind die Leitungen zu- und abflussseitig zum Filter mit Entlüftungsfiltem zu versehen. Diese Entlüftungsfilter werden während des Aufbaus der Vorrichtung über Verschlüsse vom Leitungsnetz getrennt, um eine Benetzung mit Flüssigkeit zu vermeiden. Während der Filtration werden die Filter zumindest auf der maximalen Höhe des Füllstands des Quellbehälters angeordnet, um eine innenseitige Benetzung zu vermeiden. Maßnahmen zur Sterilisation oder sterilen Verwendung solcher Vorrichtungen werden hier nicht erläutert.

Aus der EP-A-1 671 664 ist ein lineares Filtersystem bekannt, bei dem über einen Filter mit Einlass und Auslass ein Blutbehandlungskreislauf bereitgestellt wird. In Leitungssysteme fest impfementierte Inline-Filter werden hier als nachteilig angesehen, da diese nach-Benutzung der Leitung stets entsorgt werden müssen, auch wenn der Filter sich im Nachhinein als unnötig herausstellt. Zudem wird das dabei notwendige Sterilisieren ganzer Baugruppen ob seiner Anforderungen an Größe und Umfang der notwendigen Sterilisation als nachteilig angesehen, da viele Filtermaterialien übliche Sterilisationsmethoden nicht vertragen. Vor diesem Hintergrund schlägt dieses Dokument vor, die Baugruppen - insbesondere den linearen Falter - zunächst einzeln und spezifisch angepasst zu sterilisieren, um anschließend Filter und Filtrationskreisläufe jeweils nach Bedarf über spezielle, aseptische Verbindungs-Verfahren zusammenzuschließen.

Nachteilig ist hierbei, dass sicheres, aspetisches Verbinden und Aufbauen von solchen Blutreinigungsanlagen qualifiziertes Personal erfordert, welches darin geübt ist, die notwendigen Vorsichtsmaßnahmen bei der Verbindung und dem Aufbau des benötigten Leitungsnetzes zu treffen. Dies macht die Verwendung modularer Systeme anspruchsvoll und zeitaufwändig.

### Stand der Technik

Eine gattungsgemäße, fertig aufgebaute Vorrichtung ist aus der DE 197 33 407 A1 bekannt. Die Vorrichtung dient dazu, allein unter Ausnutzung der Schwerkraft in einer Filteranordnung Blut in seine zellulären Bestandteile mit Erythrozyten einerseits und Plasma mit Leukozyten andererseits aufzutrennen und in getrennten Aufnahmebehältern aufzufangen. Derartige Vorrichtungen werden zur Aufbereitung von Blut bei der Bluteigenspende aber auch zur Aufbereitung von Blut im Rahmen von Blutspendeaktionen verwendet.

Dabei weist der Filter einen Eingang und zwei Ausgänge auf, von denen ein Ausgang die zuflusseitig in Lösung aufkonzentrierten, zellulären Bestandteile abführt, während der andere Ausgang abflussseitig zum Filter das Blutplasma abführt. Luft- und keimdicht in einem Gehäuse angeordnet erlaubte ein solcher Filter mit durch den Filter getrennter Einlass- und Aulasskammer eine Aufkonzentrierung zellulärer, in Lösung befindlicher Bestandteile auf mindestens 62% direkt mit nur einer Filtrationsstufe, ohne dass weitere Maßnahmen wie Zentrifugieren ergriffen werden mussten.

Da gattungsgemäße Vorrichtungen mit menschlichen Körpern in Verbindung gebracht werden, muss eine solche Vorrichtung sterilisiert sein. Ein häufig angewendetes Verfahren ist hierbei die Dampfsterilisation. Bei der Dampfsterilisation wird feuchter Dampf mit einer Temperatur von ca. 120 bis 135 °C auf das zu sterilisierende Objekt geleitet. Dies führt bei der gattungsgemäßen Vorrichtung zur Auftrennung von Blut in einzelne und/oder Gruppen seiner Bestandteile zu verschiedenen Problemen. So kommt es bei der Anwendung der Dampfsterilisation und den damit verbundenen Temperaturen in der bekannten Filteranordnung zur Ausbildung von Unterdruck in der mit den Filterelementen versehenen Filteranordnung mit Einlass- und Auslasskammer. Dies führt dazu, dass aus den angeschlossenen Schläuchen die Luft heraus gesaugt wird und diese kollabieren und aneinander kleben. Dies wiederum kann die spätere Einsatzfähigkeit und Benutzbarkeit der gesamten Vorrichtung beeinträchtigen, da die Schläuche gegebenenfalls auch dauerhaft deformiert werden. Ausserdem ist bei der vorbekannten Vorrichtung keine Möglichkeit vorgesehen, in das zusammengebaute, fertige System feuchten, heißen Dampf zur Dampfsterilisation einleiten zu können. Es ist daher notwendig, die einzelnen Bestandteile auf bekannte Weise innenseitig vorher separat und getrennt zur sterilisieren oder aber auf eine innenseitige Sterilisation zu verzichten und es bei einer äusserlich angewendeten Dampfsterilisation oder thermischen Sterilisation zu belassen.

Der Erfindung liegt daher die Aufgabe zugrunde, eine Lösung zu schaffen, die die umfassende Anwendung einer thermischen Sterilisation, insbesondere Dampfsterilisation, zur Sterilisation der Vorrichtung ermöglicht.

Bei einer Vorrichtung der eingangs bezeichneten Art wird diese Aufgabe gemäß den Merkmalen des Anspruches 1 gelöst. Hierbei mündet.in die eingangsseitige Leitung der Einlasskammer oder in die Einlasskammer und in die ausgangsseitige Leitung der Auslasskammer oder in die Auslasskammer jeweils eine zur Außenumgebung über einen hydrophoben Bakterienfilter in Verbindung stehende Leitung ein.

Hierbei sieht die Erfindung in vorteilhafter Weiterbildung ebenfalls vor, dass in die ausgangsseitige Leitung der Einlasskammer oder in die Einlasskammer eine zur Au-βenumgebung über einen hydrophoben Bakterienfilter in Verbindung stehende Leitung einmündet.

Hydrophobe Bakterienfilter sind wasserundurchlässige Filter mit einer geringen Porengrösse, beispielsweise im Bereich um 0,2 Mikrometer. Durch diese hydrophoben Bakterienfilter kann Gas, somit auch Wasserdampf, aber keine Flüssigkeit, insbesondere kein Wasser, hindurch treten. Mit Hilfe dieser Filter ist es bei einer Dampfsterilisation somit möglich, dass im Falle, dass sich in der Filteranordnung ein Unterdruck ausbildet, durch die mit der Filteranordnung leitungsmäßig verbundenen hydrophoben Bakterienfilter Luft von außen angesaugt und in das System eingebracht wird. Es kommt nicht zur Kollabierung der Leitungen, da ein permanenter Druckausgleich erfolgt. Ausserdem ist die Sterilisation weiterhin erfolgreich durchzuführen, da die hydrophoben Bakterienfilter mit einer Porenöffnung von 0,2 Mikrometern als Bakterienfilter wirken und von außen keine Bakterien in die mit dem jeweiligen Filter versehene Leitung eintreten können.

Mit Hilfe der hydrophoben Bakterienfilter ist es nun aber darüber hinaus auch möglich, zur thermischen Sterilisation im Rahmen der Dampfsterilisation Dampf von au-βen durch die hydrophoben Filter in die mit dem jeweiligen Filter versehene Leitung und damit ins Innere der Vorrichtung einzubringen. Es ist damit möglich, also auch die Innenseite der Vorrichtung zur Auftrennung von Blut in seine Bestandteile und/oder Gruppen seiner Bestandteile mittels Dampfsterilisation zu beaufschlagen und zu sterilisieren.

Insgesamt wird mit der Erfindung somit die Möglichkeit geschaffen, die Gesamtvorrichtung nach dem Zusammenbau und Zusammenstellung der einzelnen Komponenten, die eine Filteranordnung, einen Aufnahmebehälter für zelluläre Blutbestandteile mit Erythrozyten, einen Aufnahmebehälter für das Blutplasma mit Leukozyten, aber auch einen Leukozytendepletionsfilter und einen Blutaufnahmebehälter umfassen kann, in seiner Gesamtheit von außen und von innen umfassend thermisch mittels Dampfsterilisation zu sterilisieren. Es müssen also nicht mehr die einzelnen Behälter und/oder Bestandteile einzeln sterilisiert werden. Es ist im Rahmen einer rationellen Fertigung möglich, zunächst die jeweils gewünschte Gesamtkonfiguration der Vorrichtung zusammen zu stellen und dann diese zu sterilisieren.

Damit nach erfolgter thermischer Sterilisation, hier Dampfsterilisation, das System geschlossen ist und die Gefahr des Eindringens von Keimen von ausserhalb ausgeschlossen wird, sieht die Erfindung in Ausgestaltung vor, dass jedem hydrophobem Bakterienfilter ein Leitungsverschlusselement zugeordnet ist. Nach erfolgter Dampfsterilisation werden die Verschlusselemente verschlossen, so dass dann eine Vorrichtung zur Trennung von Blut in seine Bestandteile als geschlossenes System bzw. als gegenüber der Außenumgebung abgeschlossenes System ausgebildet ist.

Hierbei ist es besonders zweckmäßig, dass das Verschlusselement in der jeweiligen Leitung näher an der Filteranordnung angeordnet ist als der jeweils zugeordnete hydrophobe Bakterienfilter, was die Erfindung ebenfalls vorsieht.

Für die Auftrennung des Blutes in seine Blutbestandteile Plasma und zelluläre Blutbestandteile ist es von Vorteil, wenn an den Filterelementen der Filteranordnung Strömungsverhältnisse herrschen, die die zellulären Blutbestandteile unbeschadet an der Außenseite der Filteranordnung entlang strömen lassen. Um dies zu erreichen, sieht die Erfindung weiterhin vor, dass die Filterelemente der Filteranordnung mit einer blutverträglichen Flüssigkeit durchtränkt sind.

Diese Ausgestaltung führt aber bei der Sterilisation zu der Problematik, dass diese Flüssigkeit verdampft, aus der Einlasskammer der Filteranordnung austritt und dann in der angeschlossenen Leitung, die gegebenenfalls einen Leukozytenabtrennfilter oder einen Leukozytendepletionsfilter enthält, kondensiert und sich niederschlägt. Das Kondensat würde dann zu einer Störung des Leukozytendepletionsfilters führen oder eine unerwünschte Hämolyse, d. h. Zerstörung, des durch den Leukozytendepletionsfilter geführten Blutes führen. Damit dies verhindert wird, sieht die Erfindung in Weiterbildung vor, dass in der eingangsseitigen Leitung der Einlasskammer, vorzugsweise in unmittelbarer Nähe der Filteranordnung, ein Verschlussventil, insbesondere ein Einwege-Bruchventil, angeordnet ist.

Die Filterelemente der Filteranordnung sind zweckmäßigerweise als Hohlfaserfilter mit Mikroporen ausgebildet und bestehen insbesondere aus Polyethersulfon oder sulfoniertem Polyethersulfon, so dass die Erfindung vorsieht, dass die Filterelemente Hohlfaserfilter, insbesondere aus Mikroporen aufweisendem Material, vorzugsweise Polyethersulfon, sind.

Um aus dem aufzutrennenden Blut Leukozyten vorab, d. h. in Strömungsrichtung vor der Filteranordnung, oder aber auch hinterher, d. h. in Strömungsrichtung nach der Filteranordnung, von entweder im Blut oder im Plasma befindlichen Leukozyten befreien zu können, sieht die Erfindung weiterhin vor, dass die Vorrichtung einen Leukozytendepletionsfilter umfasst.

Als besonders vorteilhaft hat es sich erwiesen, dass der Leukozytendepletionsfilter in der eingangsseitigen Leitung der Einlasskammer der Filteranordnung zwischen der Filteranordnung und der Blutquelle und/oder dem dieser Leitung zugeordneten hydrophoben Bakterienfilter angeordnet ist, was die Erfindung ebenfalls vorsieht.

Eine besonders gut einzusetzende Vorrichtung erhält man dann, wenn die Vorrichtung nicht nur mit einer Blutquelle verbindbar ist, sondern eine solche bereits umfasst.

Die Erfindung zeichnet sich daher weiterhin dadurch aus, dass die Vorrichtung eine Blutquelle umfasst und die Einlasskammer der Filteranordnung mit der Blutquelle verbunden ist.

Zweckmäßig ist es hierbei weiterhin, wenn die Blutquelle einen Blutaufnahmebehälter umfasst. So ist es beispielsweise möglich, venöses Blut zunächst in einem Blutaufnahmebehälter als Sammelbehälter zu sammeln und aufzunehmen, um es dann anschließed in der Filteranordnung in seine Bestandteile aufzutrennen. Gemäss Ausgestaltung der Erfindung ist daher vorgesehen, dass die Blutquelle einen Blutaufnahmebehälter umfasst.

Um eine kompakt bauende Gesamtvorrichtung zu erhalten, zeichnet sich die Erfindung dann weiterhin dadurch aus, dass eine Ausgangsleitung des Blutaufnahmebehälters mit der eingangsseitigen Leitung der Einlasskammer der Filteranordnung in Leitungsverbindung steht. Hierbei ist es dann weiterhin von Vorteil, wenn die Vorrichtung einen mit einer Zuleitung und der Ausgangsleitung versehenen Blutaufnahmebehälter umfasst, dessen Zuleitung an ihrem dem Aufnahmebehälter abgewandten Ende mit einem mit einem oder mehreren Blutgefässen eines Spenders koppelbaren Verbindungselement versehen ist, was die Erfindung ebenfalls vorsieht.

Da, wie eingangs schon erwähnt, die Erfindung insbesondere die Aufgabe löst, eine Dampfsterilisation umfassend durchführen zu können, zeichnet sich die Erfindung ebenfalls dadurch aus, dass die Vorrichtung mittels Dampfsterilisation durch Einleitung von Dampf durch die hydrophoben Bakterienfilter innenseitig dampfsterilisierbar ist. Durch die hydrophoben Bakterienfilter kann in die Zuleitungen und damit in die Vorrichtung heißer, feuchter Sterilisationsdampf eingeleitet werden und damit eine Dampfsterilisation auch auf den Innenseiten und Innenflächen der Leitungen durchgeführt werden.

Neben der Filteranordnung, die mit einer blutverträglichen Flüssigkeit durchtränkte Filterelemente aufweisen kann, können auch der Blutsammelbehälter und der die zellulären Blutbestandteile und Erythrozyten aufnehmende Aufnahmebehälter Flüssigkeiten wie einen Stabilisator für Blutkonserven oder eine Flüssigkeit zur Erythrozytenhaltbarmachung bereits zum Zeitpunkt der thermischen Sterilisation, insbesondere der Dampfsterilisation, beinhalten. Damit auch in diesem Falle eine thermische Sterilisation, insbesondere Dampfsterilisation, problemlos möglich ist, zeichnet sich die Erfindung in vorteilhafter Weise weiterhin dadurch aus, dass Aufnahmebehälter oder Filteranordnungen, die zum Zeitpunkt der Dampfsterilisation eine verdampfbare Flüssigkeit enthalten, in ihrer Verbindungsleitung zur Filteranordnung und/oder zum Leukozytenfilter ein Verschlusselement, insbesondere ein Einweg-Bruchventil, aufweisen. Hierdurch ist es möglich, beispielsweise während der Dampfsterilisation die Einweg-Bruchventile geschlossen zu halten, so dass aus den jeweiligen Aufnahmebehältern keine verdampfte Flüssigkeit austreten kann, während nach erfolgter Sterilisation und zur Inbetriebnahme der erfindungsgemässen Vorrichtung diese Ventile geöffnet, bei Einweg-Bruchventifen diese durch Aufbrechen geöffnet werden, so dass nunmehr eine Leitungsverbindung zwischen Filteranordnung und dem jeweiligen Aufnahmebeutel besteht.

Bei Einweg-Bruchventilen handelt es sich um so genannte Inline-Ventile, die vollkommen von der jeweiligen röhrenförmigen, üblicherweise aus Kunststoff bestehenden, Zuleitung umhüllt in der Leitung angeordnet sind, so dass sie von außen nicht unmittelbar zugänglich sind. Derartige Ventile bestehen aus ineinander schiebbaren Elementen, die durch Ineinanderschieben der Elemente geschlossen werden, was bei flexiblen Kunststoffschläuchen dadurch möglich ist, dass von außen die Kunststoffschläuche erfasst werden. Geöffnet werden diese Ventile durch kurzes Aufbrechen, was ebenfalls durch Erfassen der flexiblen Kunststoffzuleitungen von Hand möglich ist.

Um die gesamte Vorrichtung besonders leicht und kompakt ausführen zu können, sieht die Erfindung weiterhin vor, dass die Aufnahmebehälter beutelförmig und/oder beutelartig ausgebildet sind.

Weiterhin weisen die verschiedenen, in der Vorrichtung zu einem Gesamtsystem zusammengefassten Einrichtungen, Elemente, Behälter, etc. eine solche Dimensionierung und einen solchen Abstand zueinander auf, dass mit der gesamten Vorrichtung eine Auftrennung von Blut in seine Bestandteile allein mit Hilfe der Schwerkraft möglich ist und erfolgt. Die Erfindung sieht daher weiterhin vor, dass die Aufnahmebeutel und de Filteranordnung in einem solchen Abstand zueinander angeordnet sind, dass die Auftrennung des Blutes ausschliesslich durch Schwerkraft erfolgt.

Schließlich wird die oben stehende Aufgabe auch durch ein Verfahren zur Dampfsterilisation einer erfindungsgemäßen Vorrichtung gemäß der Merkmale des Verfahrensanspruchs 17 gelöst, die sich unter anderem dadurch auszeichnet, dass durch die hydrophoben Bakterienfilter Sterilisationsdampf in die Vorrichtung eingeleitet wird. Durch die Ausbildung von hydrophoben Bakterienfiltern an den zu oder von der Filteranordnung führenden schlauchförmigen Leitungen aus Kunststoffschlauch ist es möglich, mittels Dampfsterilisation das System bzw. die Vorrichtung auch von innen bzw. innenseitig zu sterilisieren. Weitere Vorteile dieses erfindungsgemässen Verfahrens sind entsprechend den Vorteilen der vorteilhaften Vorrichtungen zugänglich.

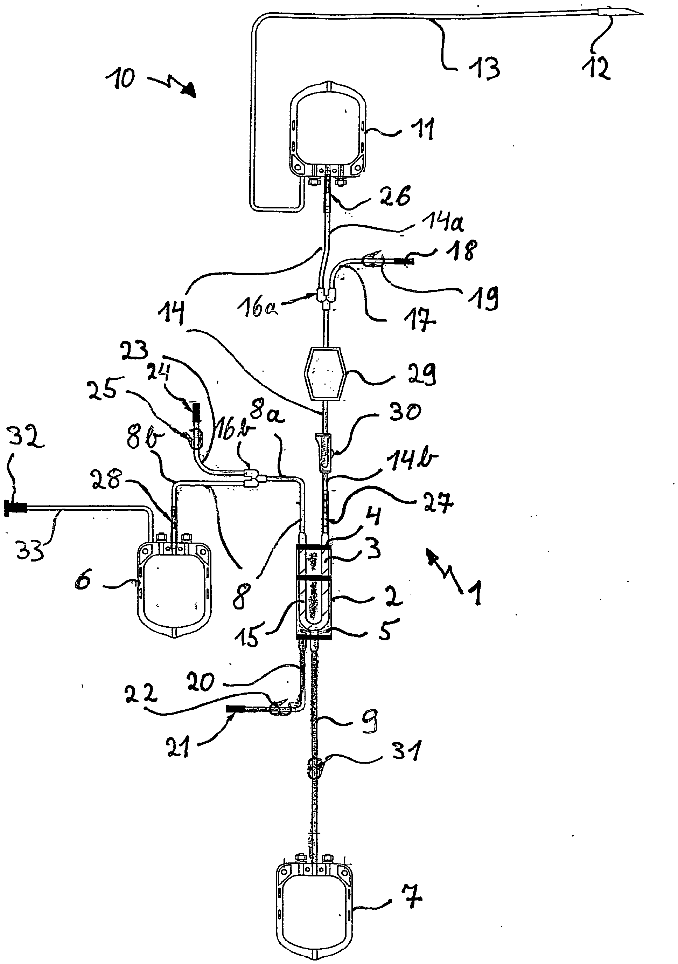

Die Erfindung ist nachstehend anhand der einzigen Figur beispielhaft näher erläutert. Diese zeigt in schematischer Gesamtdarstellung die insgesamt mit 1 bezeichnete Vorrichtung zur Auftrennung von Blut in einzelne und/oder Gruppen seiner Bestandteile in Form eines sterilisierbaren Systems. Die Vorrichtung umfasst eine Filteranordnung 2 , die mittels darin angeordneter Filterelemente 3 eine Einlasskammer 4 und eine Auslasskammer 5 ausbildet. Weiterhin umfasst die Vorrichtung 1 einen Aufnahmebehälter 6 zur Aufnahme zellulärer, in der Filteranordnung 2 abgetrennter Blutbestandteile, die Erythrozyten umfassen, sowie einen Aufnahmebehälter 7 für in der Filteranordnung 2 abgetrenntes Blutplasma, das die Leukozyten umfasst. Die Einlasskammer 4 der Filteranordnung 2 ist ausgangsseitig mittels einer als Kunststoffschlauch ausgeführten, aus den Leitungsabschnitten 8a und 8b bestehenden Leitung 8 mit dem Aufnahmebehälter 6 für zelluläre Bestandteile des Blutes leitungsmäßig verbunden. Die Auslasskammer 5 der Filteranordnung 2 ist ausgangsseitig mittels einer aus einem Kunststoffschlauch gebildeten Leitung 9 mit dem Aufnahmebehälter 7 für das Blutplasma leitungsmäßig verbunden.

Die Vorrichtung 1 umfasst weiterhin eine insgesamt mit 10 bezeichnete Blutquelle, die einen Blutaufnahmebehälter 11 und eine Einstechnadel 12 umfasst, die über eine Leitung 13 mit dem Blutaufnahmebehälter 11 verbunden ist. In dem Blutaufnahmebehälter 11 kann somit venöses oder arterielles Blut gesammelt und als Blutquelle zur Verfügung gestellt werden. Es ist aber auch möglich, an dieser Stelle lediglich bereits von einer Blutspende stammendes und in einem beutelartigen Aufnahmebehälter 11 befindliches Blut als Blutquelle 10 zur Verfügung zu stellen. Der Blutaufnahmebehälter 11 ist ausgangsseitig mittels einer die Leitungsabschnitte 14a und 14b umfassenden, von einem Kunststoffschlauch gebildeten Leitung 14 mit der Eingangsseite der Einlasskammer 4 der Filteranordnung 2 verbunden.

Die Aufnahmebehälter 6 , 7 und 11 sind beutelförmig oder beutelartig ausgebildet und wie bekannte Bluttransfusionsbeutel flexibel in Bezug auf ihre Stabilität. Im Aufnahmebeutel 6 befindet sich vor Durchführung der Blutauftrennung und auch vor Durchführung einer Sterilisation des Gesamtsystems bzw. der Gesamtvorrichtung 1 eine verdampfbare Flüssigkeit zur Erythrozytenhaltbarmachung (Mannitol) und im Aufnahmebeutel 11 befindet sich ein flüssiger, ebenfalls verdampfbarer Stabilisator für Blutkonserven. Ebenso sind die Hohlfaserfilter 15, die Mikroporen ausbilden und insbesondere aus Polyethersulfon bestehen, mit einer blutverträglichen Flüssigkeit durchtränkt.

In die eingangsseitige Leitung 14 der Einlasskammer 4 mündet mittels eines dreiendigen Verbindungselementes 16a eine von einem Kunststoffschlauch gebildete Leitung 17 ein, die an ihrer dem dreiendigen Verbindungselement 16a abgewandten Seite über einen hydrophoben Bakterienfilter 18 mit der Außenumgebung in Verbindung steht. Zwischen dem hydrophoben Bakterienfilter 18 und dem dreiendigen Verbindungselement 16a ist in der Leitung 17 ein Leitungsverschlusselement 19 ausgebildet.

Ebenso mündet in die Auslasskammer 5 der Filteranordnung 2 eine von einem Kunststoffschlauch gebildete Leitung 20 ein, die an ihrem der Auslasskammer 5 abgewandten Ende einen hydrophoben Bakterienfilter 21 aufweist, über den die Leitung 20 mit der Außenumgebung in Verbindung steht. Weiterhin weist auch die Leitung 21 ein Leitungsverschlusselement 22 auf. Auch in die ausgangsseitige Leitung 8 der Einlasskammer 4 der Filteranordnung 2 mündet mittels eines dreiendigen Verbindungselementes 16b eine Leitung 23 ein, die an ihrem dem dreiendigen Verbindungselement 16b abgewandten Ende mit einem weiteren hydrophoben Bakterienfilter 24 versehen ist und über diesen hydrophoben Bakterienfilter 24 mit der Außenumgebung in Verbindung steht. Zwischen dem hydrophoben Bakterienfilter 24 und dem dreiendigen Verbindungselement 16b ist in der Leitung 23 ein Leitungsverschlusselement 25 ausgebildet.

In der Leitung 14 ist sowohl in ihrem Endbereich im Bereich des Blutaufnahmebehälters 11 als auch in ihrem Endbereich im Bereich der Einlasskammer 4 der Filteranordnung 2 jeweils ein innerhalb der Schlauchleitung 14 befindliches Ventil, insbesondere ein Einwege-Bruchventil 26 , 27 angeordnet. Ein weiteres Ventil 28 , insbesondere auch in diesem Fall eine Einwege-Bruchventil, ist in der Leitung 8 in der Nähe ihres Endes im Bereich des Aufnahmebehälters 6 ebenfalls innerhalb der Schlauchleitung 8 befindlich angeordnet.

In der Leitung 14 ist zwischen Blutaufnahmebehälter 11 und der Filteranordnung 2 und auch zwischen der Filteranordnung 2 und dem dreiendigen Verbindungselement 16a und somit der Einmündung der Leitung 17 in die Leitung 14 ein Leukozytentrennfilter bzw. ein Leukozytendepletionsfilter 29 angeordnet. Weiterhin umfasst die Leitung 14 ein Verstellelement 30 , mit dessen Hilfe die Fliessgeschwindigkeit der in der Leitung 14 zur Filteranordnung 2 fliessenden Flüssigkeit reguliert werden kann. Ein Leitungsverschlusselement 31 ist zudem in der zum Aufnahmebehälter 7 führenden Leitung 9 angeordnet und schliesslich weist der Aufnahmebehälter 6 ausgangsseitig eine mit einem Stopfen 32 verschlossene Leitung 33 auf.

Mit Hilfe der Vorrichtung 1 lässt sich allein durch Schwerkraft aus dem Blutaufnahmebehälter 11 entnommenes Blut in der Filteranordnung 2 in Blutplasma mit Leukozyten auftrennen, das dann über die Auslasskammer 5 der Filteranordnung 2 in dem Aufnahmebehälter 7 aufgefangen wird, und in zelluläre Blutbestandteile mit Erythrozyten auftrennen, die dann über die ausgangsseitige Leitungsverbindung 8 der Einlasskammer 4 in dem Aufnahmebehälter 6 aufgefangen werden. Eine solche Vorrichtung muss vor Inbetriebnahme und Gebrauch am menschlichen Körper sterilisiert sein. Damit diese Vorrichtung 1 sterilisierbar ist, ist sie mit hydrophoben Bakterienfiltern 18 , 21 und 24 versehen, die jeweils leitungsmäßig derart mit der Einlasskammer 4 und/oder der Auslasskammer 5 der Filteranordnung 2 verbunden sind, das alle Eingangs- und Ausgangsseiten und damit der gesamte Innenbereich der Filteranordnung 2 , aber auch die an der jeweiligen Eingangs- und/oder Ausgangsseite der Filteranordnung 2 angeschlossenen Leitungen 8 , 9 und 14 sowie die an dem der Filteranordnung 2 abgewandten Ende dieser Leitungen 8 , 9 und 14 jeweils angeschlossenen Aufnahmebehälter 6 , 7 und 11 von durch die hydrophoben Bakterienfilter in die angeschlossenen Leitungen eingeleitetem heißen, feuchten Sterilisationsdampf erreicht werden bzw. erreichbar sind. Damit ist es möglich, mit dieser Ausgestaltung die gesamten Innenwandungen und Innenflächen der Vorrichtung 1 mit Hilfe einer Dampfsterilisation zu sterilisieren. Nachdem eine Dampfsterilisation erfolgt ist, sind die die hydrophoben Bakterienfilter aufweisenden Leitungen 17 , 20 und 23 mittels der darin angeordneten Leitungsverschlusselemente 19, 22 und 25 verschließbar, so dass anschließed keine Umgebungsluft mehr in das System durch diese Bleitungen eindringen kann.

Weiterhin ist es möglich, vor Durchführung einer von außen auf die Elemente und Bestandteile sowie Vorrichtungen und Einrichtungen der Vorrichtung 1 einwirkenden Dampfsterilisation in den Leitungen 14 und 8 ummittelbar an den daran angeschlossenen Aufnahmebehältern 6 und 11 bzw. der Filteranordnung 2 befindliche Ventile 26 , 27 und 28 zu verschließe, so dass in den Aufnahmebehältern 6 und 11 sowie der Filteranordnung 2 befindliche Flüssigkeit nicht aus den Aufnahmebehältern 6, 11 und der Filteranordnung 2 in die angeschlossenen Leitungen austreten und dort kondensieren kann.

In Fällen, in denen durch das gleichzeitige Eintreten von Sterilisationsdampf in das System nicht sichergestellt ist, dass nicht doch in den Behältern 6, 11 und der Filteranordnung 2 befindliche Flüssigkeit verdampfen und austreten kann, werden die Ventile 26 , 27 und 28 vor Beginn des Sterilisationsverfahrens geschlossen. Eine Sterilisation der Behälter 6 und 11 erfolgt dann lediglich durch von außen auf die Behälter einwirkende Dampfsterilisation. Die übrigen Leitungsbereiche werden durch die über die hydrophoben Bakterienfilter 18 , 21 und 24 einleitbaren Sterilisationsdampf auch von innen sterilisiert.

Es erfolgt eine vorteilhafte Dampfsterilisation weiterhin dadurch, dass zunächst die Einweg-Bruchventile 26 , 27 und 28 geschlossen sind und dann die Dampfsterilisation, d. h. die Beaufschlagung des Systems mit heißem, feuchtem Sterilisationsdampf im Temperaturbereich von 120 bis 135 °C durchgeführt wird. Durch diese Sterilisation wird die Gesamtvorrichtung ausreichend sterilisiert. Aus den Behältern 6 und 11 kann darin eventuell vorhandene Flüssigkeit nicht austreten, da die Ventile 26 und 28 geschlossen sind. Ebenso kann aus der Filteranordnung 2 keine Flüssigkeit bzw. verdampfte Flüssigkeit in die Leitung 14 austreten. Ein Kollabieren der Leitungen 14 , 8 und 20 sowie 9 ist dadurch verhindert, dass die Sterilisation bei geöffneten Verschlüssen 19 , 22 und 25 sowie gegebenenfalls 31 durchgeführt wird, so dass die entsprechenden Leitungen über die hydrophoben Bakterienfilter 18 , 21 und 24 von der Außenumgebung Luft in die Leitungen einsaugen können, sobald sich in der Filteranordnung 2 ein Unterdruck einstellt.

Nach durchgeführter Sterilisation werden die Verschlusselemente 19 , 22 und 25 geschlossen. Wenn dann der Prozess zur Bluttrennung durchgeführt werden soll, werden die Einweg-Bruchventile 26 , 27 und 28 durch Aufbrechen geöffnet, so dass nun Blut aus dem Blutaufnahmebehälter 11 in die Leitung 14 austreten und nach Durchlaufen des Leukozytendepletionsfilters 29 in die Einlasskammer 4 eintreten und mittels der Filterelemente 3 in die im Aufnahmebehälter 6 aufgefangenen zellulären Blutbestandteile und die im Aufnahmebehälter 7 aufgefangenen Plasmabestandteile aufgetrennt werden. Hierbei verbleiben die zellulären Blutbestandteile auf der der Einlasskammer zugewandten Seite der Filterelemente, wohingegen das Blutplasma durch die Poren der Filterelemente hindurch auf die der Auslasskammer zugewandte Seite der Filterelemente hindurch treten.

Wenn der Blutaufnahmebeutel 11 geleert ist, wird dann der Verschluss 19 wiederum geöffnet, so dass im Leukozytendepletionsfilter ein Druckausgleich stattfinden kann, der auch letzte, noch im Leukozytendepletionsfilter 29 befindliche Blutreste in die Filteranordnung 2 austreten lässt.

Wenn es am Ende des Bluttrennungsprozesses zu einer Luftblasenbildung im Erythrozytenbeutel 6 gekommen sein sollte, kann durch entsprechende Beaufschlagung des Beutels 6 diese Luftblase durch den hydrophoben Filter 24 ausgeblasen werden, wozu dann auch das Leitungsverschlusselement 25 wiederum geöffnet wird.

Bei den Filterelementen 3 handelt es sich um Hohlfaserfilter, die aus der Membranfiltration, insbesondere der Ultrafiltration, bekannt sind. Beispielsweise handelt es sich dabei um einen Hohlfaserfilter, der Membranen mit Mikrofasern PES-TF 10 (Firma Akzo) aus Polyethersulfon-/sulfoniertem Polyethersulfon - Material enthält bzw. aus solchem besteht. Diese Membranen weisen einen mittleren Innendurchmesser von 300 Mikrometern, eine mittlere Wanddicke von 100 Mikrometern und eine maximale Porengrösse von 0,5 Mikrometern auf. Die Hohlfaserfilter sind als röhrenförmige Einzelelemente so nebeneinander angeordnet, dass die Gesamtheit der Innenbereiche der Hohlfasern die Einlasskammer 4 und die Gesamtheit der Außenbereiche der Hohlfasern die Auslasskammern 5 ausbildet. Nach durchgeführter Dampfsterilisation oder thermischer Sterilisation sind die an die Einlasskammer 4 und die Auslasskammer 5 der Filteranordnung 2 angeordneten Leitungen alle luftdicht und keimdicht mit den Aufnahmebehältern 6 für zelluläre Blutbestandteile und 7 für Blutplasma sowie dem Blutaufnahmebehälter 11 verbunden.

## Patentansprüche

1. Vorrichtung (1) zur Auftrennung von Blut in einzelne und/oder Gruppen seiner Bestandteile in Form eines Systems, das System als zusammengebaute Gesamtvorrichtung umfassend
- eine Filteranordnung (2), die mittels darin angeordneter Filterelemente (3)
- eine Einlasskammer (4) und
- eine Auslasskammer 5) ausbildet,
- einen Aufnahmebehälter (6) für zelluläre Blutbestandteile und
- einen Aufnahmebehälter (7) für Blutplasma,
wobei die Einlasskammer (4) der Filteranordnung (2) eingangsseitig mittels einer von einem Kunststoffschlauch gebildeten Leitung (14) mit einer Blutquelle (10) leitungsmäßig verbindbar ist,
- die Einlasskammer (4) ausgangsseitig mittels einer aus Kunststoffschlauch ausgeführten Leitung (8) mit dem Aufnahmebehälter (6) für zelluläre Bestandteile des Blutes verbunden ist und
- die Auslasskammer (5) ausgangsseitig mittels einer aus Kunststoffschlauch gebildeten Leitung (9) mit dem Aufnahmebehälter (7) für Blutplasma leitungsmäßig verbunden ist,
**dadurch gekennzeichnet,**
**dass** in die eingangsseitige Leitung (14) der Einlaßkammer (4) oder in die Einlasskammer (4) und in die ausgangsseitige Leitung (9) der Auslasskammer (5) oder in die Auslasskammer (5) jeweils eine zur Außenumgebung über einen hydrophoben Bakterienfilter (18, 21) in Verbindung stehende Leitung (17, 20) einmündet,
wobei 'hydrophob' bedeutet, dass der Bakterienfilter (18, 21)
- ein wasserundurchlässiger Filter mit geringer Porengröße ist
- ein gas- und wasserdampfdurchlässiger Filter ist
und
die Gesamtvorrichtung mittels Dampfsterilisation durch Einleitung von heißem, feuchtem Dampf
durch die Bakterienfilter (18, 21, 24)
in die Zuleitungen und damit in die Vorrichtung
innenseitig dampfsterilisierbar ausgebildet ist.

2. Vorrichtung nach Anspruch 1, **dadurch gekennzeichnet, dass** in die ausgangsseitige Leitung ( 8 ) der Einlasskammer ( 4 ) oder in die Einlasskammer ( 4 ) eine zur Au-βenumgebung über einen hydrophoben Bakterienfilter ( 24 ) in Verbindung stehende Leitung ( 23 ) einmündet.

3. Vorrichtung nach Anspruch 1 oder 2, **dadurch gekennzeichnet, dass** jedem hydrophobem Bakterienfilter ( 18, 21 , 24 ) ein Leitungsverschlusselement ( 19 , 22 , 25 ) zugeordnet ist.

4. Vorrichtung nach Anspruch 3, **dadurch gekennzeichnet, dass** das Leitungsverschlusselement ( 19, 22, 25 ) in der jeweiligen Leitung ( 17, 20 , 23 ) näher an der Filteranordnung ( 2 ) angeordnet ist als der jeweils zugeordnete hydrophobe Bakterienfilter ( 18, 21, 24).

5. Vorrichtung nach einem der vorhergehenden Ansprüche, **dadurch gekennzeichnet, dass** die Filterelemente ( 3 ) der Filteranordnung ( 2) mit einer blutverträglichen Flüssigkeit durchtränkt sind.

6. Vorrichtung nach einem der vorhergehenden Ansprüche, **dadurch gekennzeichnet, dass** in der eingangsseitigen Leitung ( 14 ) der Einlasskammer ( 4 ), vorzugsweise in unmittelbarer Nähe der Filteranordnung ( 2 ), ein Verschlussven6l ( 27 ), insbesondere ein Einwege-Bruchventil, angeordnet ist.

7. Vorrichtung nach einem der vorhergehenden Ansprüche, **dadurch gekennzeichnet, dass** die Filterelemente ( 3) Hohlfaserfilter, insbesondere aus Mikroporen aufweisendem Material, vorzugsweise Polyethersulfon, sind.

8. Vorrichtung nach einem der vorhergehenden Ansprüche, **dadurch gekennzeichnet, dass** die Vorrichtung ( 1 ) einen Leukozytendepletionsfilter ( 29) umfasst.

9. Vorrichtung nach Anspruch 8, **dadurch gekennzeichnet, dass** der Leukozytendepletionsfilter ( 29 ) in der eingangsseitigen Leitung ( 14 ) der Einlasskammer ( 4 ) der Filteranordnung ( 2 ) zwischen der Filteranordnung ( 2 ) und der Blutquelle ( 10 ) und/oder dem dieser Leitung ( 14 ) zugeordneten hydrophoben Bakterienfilter ( 18) angeordnet ist.

10. Vorrichtung nach einem der vorhergehenden Ansprüche, **dadurch gekennzeichnet, dass** die Vorrichtung ( 1 ) eine Blutquelle ( 10 ) umfasst und die Einlasskammer ( 4 ) der Filteranordnung ( 2 ) mit der Blutquelle ( 10) verbunden ist.

11. Vorrichtung nach Anspruch 10, **dadurch gekennzeichnet, dass** die Blutquelle ( 10 ) einen Blutaufnahmebehälter ( 11) umfasst.

12. Vorrichtung nach einem der vorhergehenden Ansprüche, **dadurch gekennzeichnet, dass** die eingangsseitige Leitung (14) eine Ausgansgangsleitung ( 14a ) des Blutaufnahmebehälters ( 11 ) und eine eingangsseitige Leitung ( 14b ) der Einlasskammer ( 4 ) umfasst, die mit der Filteranordnung ( 2) in Leitungsverbindung steht.

13. Vorrichtung nach einem der Ansprüch 1-10, **dadurch gekennzeichnet, dass** sie einen mit einer Zuleitung ( 13 ) und einer Ausgangsleitung ( 14a ) versehenen Blutaufnahmebehälter ( 11) auf weist, dessen Zuleitung ( 13 ) an ihrem den Aufnahmebehälter ( 11 ) abgewandten Ende mit einem mit einem oder mehreren Blutgefässen eines Spenders koppelbaren Verbindungselement ( 12) versehen ist.

14. Vorrichtung nach einem der vorhergehenden Ansprüche, **dadurch gekennzeichnet, dass** die Aufnahmebehälter ( 6 , 11 ) oder die Filteranordnung ( 2 ), die zum Zeitpunkt der Dampfsterilisation eine verdampfbare Flüssigkeit enthalten, in ihrer jeweiligen Verbindungsleitung ( 8 , 14 ) zur Filteranordnung ( 2 ) und/oder zum Leukozytendepletionsfilter ( 29 ) ein Verschlusselement ( 26 , 27 , 28), insbesondere ein Einweg-Bruchventil, aufweisen.

15. Vorrichtung nach einem der vorhergehenden Ansprüche, **dadurch gekennzeichnet, dass** die Aufnahmebehälter ( 6, 7, 11) beutelförmig und/oder beutelartig ausgebildet sind.

16. Vorrichtung nach Anspruch 15, **dadurch gekennzeichnet, dass** die Aufnahmebeutel ( 6 , 7 , 11 ) und die Filteranordnung ( 2) in einem solchen Abstand zueinander angeordnet sind, dass die Auftrennung des Blutes ausschliesslich durch Schwerkraft erfolgt.

17. Verfahren zur Dampfsterilisation einer Vorrichtung ( 1) zur Auftrennung von Blut in einzelne und/oder Gruppen seiner Bestandteile , wobei
eine Gesamtvorrichtung gemäß einem der Ansprüche 1-16 als System zusammengebaut und zusammengestellt wird und die Gesamtvorrichtung in ihrer Gesamtheit mit heißem, feuchtem Dampf sterilisiert wird, wobei durch die hydrophoben Bakterienfilter ( 18, 21 , 24 ) Sterilisationsdampf in die Vorrichtung (1) eingeleitet wird.

## Claims

1. Device (1) for fractionating blood into single ones and/or groups of its components in the form of a system, the system, as assembled complete device, comprising
- a filter arrangement (2) which, by means of filter elements (3) dispersed therein, forms
- an inlet chamber (4) and
- an outlet chamber (5),
- a receptacle (6) for cellular blood components and
- a receptacle (7) for blood plasma,
where the inlet chamber (4) of the filter arrangement (2) can be connected by conduit on the entry side by means of a conduit (14) formed by a plastic tubing to a blood source (10),
- the inlet chamber (4) is connected on the exit side by means of a conduit (8) made from plastic tubing to the receptacle (6) for cellular components of the blood, and
- the outlet chamber (5) is connected by conduit on the exit side by means of a conduit (9) formed from plastic tubing to the receptacle (7) for blood plasma,
**characterized in that**
in each case a conduit (17, 20) which is connected to the external environment through a hydrophobic bacterial filter (18, 21) opens into the conduit (14), on the entry side, of the inlet chamber (4) or into the inlet chamber (4) and into the conduit (9), on the exit side, of the outlet chamber (5) or into the outlet chamber (5),
wherein "hydrophobic" means that the bacterial filter (18, 21)
- is a water-impermeable filter having a small pore size
- is a gas-permeable and water-vapour-permeable filter and
the complete device is steam-sterilizable on the inside by means of steam sterilization by passing
hot, moist steam
through the bacterial filters (18, 21, 24)
into the feed conduits and hence into the device.

2. Device according to Claim 1, **characterized in that** a conduit (23) which is connected to the external environment through a hydrophobic bacterial filter (24) opens into the conduit (8) on the exit side of the inlet chamber (4) or into the inlet chamber (4).

3. Device according to Claim 1 or 2, **characterized in that** each hydrophobic bacterial filter (18, 21, 24) is associated with a conduit closure element (19, 22, 25).

4. Device according to Claim 3, **characterized in that** the conduit closure element (19, 22, 25) is disposed in the respective conduit (17, 20, 23) closer to the filter arrangement (2) than to the respective associated hydrophobic bacterial filter (18, 21, 24).

5. Device according to any of the preceding claims, **characterized in that** the filter elements (3) of the filter arrangement (2) are impregnated with a blood-compatible liquid.

6. Device according to any of the preceding claims, **characterized in that** a closure valve (27), in particular a disposable rupture valve, is disposed in the conduit (14) on the entry side of the inlet chamber (4), preferably in the direct vicinity of the filter arrangement (2).

7. Device according to any of the preceding claims, **characterized in that** the filter elements (3) are hollow fiber filters, in particular made of material having micropores, preferably polyethersulfone.

8. Device according to any of the preceding claims, **characterized in that** the device (1) comprises a leukocyte depletion filter (29).

9. Device according to Claim 8, **characterized in that** the leukocyte depletion filter (29) is disposed in the conduit (14) on the entry side of the inlet chamber (4) of the filter arrangement (2) between the filter arrangement (2) and the blood source (10) and/or the hydrophobic bacterial filter (18) associated with this conduit (14).

10. Device according to any of the preceding claims, **characterized in that** the device (1) comprises a blood source (10), and the inlet chamber (4) of the filter arrangement (2) is connected to the blood source (10).

11. Device according to Claim 10, **characterized in that** the blood source (10) comprises a blood receptacle (11).

12. Device according to any of the preceding claims, **characterized in that** the conduit (14) on the entry side comprises an exit conduit (14a) of the blood receptacle (11) and a conduit (14b) on the entry side of the inlet chamber (4) which is connected by conduit to the filter arrangement (2).

13. Device according to any of Claims 1-10, **characterized in that** it has a blood receptacle (11) which is provided with a feed conduit (13) and an exit conduit (14a), the feed conduit (13) of which is provided at its end facing away from the receptacle (11) with a connecting element (12) which can be coupled to one or more blood vessels of a donor.

14. Device according to any of the preceding claims, **characterized in that** the receptacles (6, 11) or the filter arrangement (2) which comprise a vaporizable liquid at the time of steam sterilization include a closure element (26, 27, 28), in particular a disposable rupture valve, in their respective connecting conduit (8, 14) to the filter arrangement (2) and/or to the leukocyte depletion filter (29).

15. Device according to any of the preceding claims, **characterized in that** the receptacles (8, 7, 11) are configured in the form of bags and/or bag-like.

16. Device according to Claim 15, **characterized in that** the receiving bags (6, 7, 11) and the filter arrangement (2) are disposed at a distance from one another such that the fractionation of blood takes place exclusively through gravity.

17. Method for steam sterilization of a device (1) for fractionating blood into single ones and/or groups of its components, wherein
a complete device according to any of Claims 1-16 is assembled and put together as a system and the complete device is sterilized in its entirety with hot, moist steam, wherein sterilization steam is passed into the device (1) through the hydrophobic bacterial filters (18, 21, 24).

## Revendications

1. Dispositif (1) pour la séparation de sang en ses composants individuels et/ou en groupes de ses composants sous la forme d'un système, le système comprenant sous forme de dispositif global assemblé:
- un dispositif de filtrage (2), qui forme, au moyen d'éléments de filtre (3) disposés dans celui-ci,
- une chambre d'entrée (4) et
- une chambre de sortie (5),
- un récipient de réception (6) pour des composants cellulaires du sang et
- un récipient de réception (7) pour du plasma sanguin,
dans lequel la chambre d'entrée (4) du dispositif de filtrage (2) peut être raccordée côté entrée en mode d'écoulement à une source de sang (10) au moyen d'une conduite (14) formée par un tuyau en matière plastique,
- la chambre d'entrée (4) est raccordée côté sortie en mode d'écoulement au récipient de réception (6) pour des composants cellulaires du sang au moyen d'une conduite (8) formée par un tuyau en matière plastique,
- la chambre de sortie (5) est raccordée côté sortie en mode d'écoulement au récipient de réception (7) pour du plasma sanguin au moyen d'une conduite (9) formée par un tuyau en matière plastique,
**caractérisé en ce qu'**il débouche respectivement dans la conduite côté entrée (14) de la chambre d'entrée (4) ou dans la chambre d'entrée (4) et dans la conduite côté sortie (9) de la chambre de sortie (5) ou dans la chambre de sortie (5) une conduite (17, 20) se trouvant en communication avec l'atmosphère extérieure via un filtre bactérien hydrophobe (18, 21),
dans lequel "hydrophobe" signifie que le filtre bactérien (18, 21)
- est un filtre imperméable à l'eau avec une petite taille de pores,
- un filtre perméable au gaz et à la vapeur d'eau,
et
le dispositif global est réalisé côté intérieur de façon stérilisable à la vapeur au moyen d'une stérilisation à la vapeur par introduction de vapeur chaude humide à travers les filtres bactériens (18, 21, 24) dans les conduites d'arrivée et ainsi dans le dispositif.

2. Dispositif selon la revendication 1, **caractérisé en ce qu'**il débouche dans la conduite côté sortie (8) de la chambre d'entrée (4) ou dans la chambre d'entrée (4) une conduite (23) se trouvent en communication avec l'atmosphère extérieure via un filtre bactérien( 24).

3. Dispositif selon la revendication 1 ou 2, **caractérisé en ce qu'**un élément de fermeture de conduite (19, 22, 25) est associé à chaque filtre bactérien hydrophobe (18, 21, 24).

4. Dispositif selon la revendication 3, **caractérisé en ce que** l'élément de fermeture de conduite (19, 22, 25) est disposé dans la conduite respective (17, 20, 23) plus près du dispositif de filtrage (2) que le filtre bactérien hydrophobe respectivement associé (18, 21,24).

5. Dispositif selon l'une quelconque des revendications précédentes, **caractérisé en ce que** les éléments de filtre (3) du dispositif de filtrage (2) sont imprégnés d'un liquide compatible avec le sang.

6. Dispositif selon l'une quelconque des revendications précédentes, **caractérisé en ce qu'**une soupape de fermeture (27), en particulier une soupape de rupture à usage unique, est disposée dans la conduite côté entrée (14) de la chambre d'entrée (4), de préférence à proximité immédiate du dispositif de filtrage (2).

7. Dispositif selon l'une quelconque des revendications précédentes, **caractérisé en ce que** les éléments de filtre (3) sont des filtres à fibres creuses, en particulier en un matériau présentant des micro-pores, de préférence un polysulfone.

8. Dispositif selon l'une quelconque des revendications précédentes, **caractérisé en ce que** le dispositif (1) comprend un filtre de déplétion des leucocytes (29).

9. Dispositif selon la revendication 8, **caractérisé en ce que** le filtre de déplétion des leucocytes (29) est disposé dans la conduite côté entrée (14) de la chambre d'entrée (4) du dispositif de filtrage (2) entre le dispositif de filtrage (2) et la source de sang (10) et/ou le filtre bactérien hydrophobe (18) associé à cette conduite (14).

10. Dispositif selon l'une quelconque des revendications précédentes, **caractérisé en ce que** le dispositif (1) comprend une source de sang (10) et la chambre d'entrée (4) du dispositif de filtrage (2) est raccordée à la source de sang (10).

11. Dispositif selon la revendication 10, **caractérisé en ce que** la source de sang (10) comprend un récipient de réception de sang (11).

12. Dispositif selon l'une quelconque des revendications précédentes, **caractérisé en ce que** la conduite côté entrée (14) comprend une conduite de sortie (14a) du récipient de réception de sang (11) et une conduite côté entrée (14b) de la chambre d'entrée (4), qui est raccordée en mode d'écoulement au dispositif de filtrage (2).

13. Dispositif selon l'une quelconque des revendications 1 à 10, **caractérisé en ce qu'**il présente un récipient de réception de sang (11) muni d'une conduite d'arrivée (13) et d'une conduite de sortie (14a), dont la conduite d'arrivée (13) est munie, à son extrémité située à l'opposé du récipient de réception de sang (11), d'un élément de raccordement (12) pouvant être couplé à une ou à plusieurs poche(s) de sang d'un donneur.

14. Dispositif selon l'une quelconque des revendications précédentes, **caractérisé en ce que** les récipients de réception (6, 11) ou le dispositif de filtrage (2), qui contiennent un liquide vaporisable au moment de la stérilisation à la vapeur, présentent un élément de fermeture (26, 27, 28), en particulier une soupape de rupture à usage unique, dans leur conduite de raccordement respective (8, 14) vers le dispositif de filtrage (2) et/ou vers le filtre de déplétion des leucocytes (29).

15. Dispositif selon l'une quelconque des revendications précédentes, **caractérisé en ce que** les récipients de réception (6, 7, 11) sont réalisés en forme de poches et/ou à la manière de poches.

16. Dispositif selon la revendication 15, **caractérisé en ce que** les poches de réception (6, 7, 11) et le dispositif de filtrage (2) sont disposés à une distance telle l'un de l'autre que la séparation du sang se produise exclusivement sous l'action de la gravité.

17. Procédé pour la stérilisation à la vapeur d'un dispositif (1) pour la séparation de sang en ses composants individuels et/ou en groupes de ses composants, dans lequel un dispositif global selon l'une quelconque des revendications 1 à 16 est assemblé et composé en forme de système et on stérilise le dispositif global dans sa totalité avec de la vapeur chaude humide, dans lequel on introduit la vapeur de stérilisation dans le dispositif (1) à travers les filtres bactériens hydrophobes (18, 21, 24).
